# EUROPEAN PATENT SPECIFICATION

(11) **EP 1 404 847 B1**
(45) Date of publication and mention of the grant of the patent: **25.05.2005**
(21) Application number: 02740737.8
(22) Date of filing: 19.06.2002
(51) Int. Cl.: C12N 15/68

(54) **METHOD OF PLASMID STABILISATION BY IN VIVO DELETING THE ANTIBIOTIC RESISTENCE AND SELECTING WITH AN ESSENTIAL GENE**
METHODE ZUR PLASMIDSTABILISIERUNG DURCH IN VIVO DELETION DER ANTIBIOTIKARESISTENZ UND SELEKTION MIT EINEM ESSENTIELLEN GEN
METHODE DE STABILISATION D'UN PLASMIDE EN DELETANT IN VIVO LA RESISTANCE A L'ANTIBIOTIQUE ET EN SELECTIONNANT AVEC UN GENE ESSENTIEL

(30) Priority: 21.06.2001 SE 0102204
(43) Date of publication of application: 07.04.2004
(73) Proprietor: Isaksson, Leif, 752 60 Uppsala (SE); Hägg, Peter Jörgen, 182 30 Danderyd (SE); Abdulkarim, Farhad Maruf, 142 32 Skogas (SE)
(72) Inventor: Isaksson, Leif, 752 60 Uppsala (SE); Hägg, Peter Jörgen, 182 30 Danderyd (SE); Abdulkarim, Farhad Maruf, 142 32 Skogas (SE)
(74) Representative: Fagerlin, Heléne
(86) International application number: PCT/EP2002/006780
(87) International publication number: WO 2003/000881

(56) References cited:
- EP-A- 0 972 838
- NAKAYAMA K ET AL: "CONSTRUCTION OF AN ASD+ EXPRESSION-CLONING VECTOR: STABLE MAINTAINANCE AND HIGH LEVEL EXPRESSION OF CLONED GENES IN A SALMONELLA VACCINE STRAIN" BIO/TECHNOLOGY, NATURE PUBLISHING CO. NEW YORK, US, vol. 6, no. 6, 1 June 1988 (1988-06-01), pages 693-697, XP000578379 ISSN: 0733-222X
- GALAN J E ET AL: "CLONING AND CHARACTERIZATION OF THE ASD GENE OF SALMONELLA TYPHIMURIUM: USE IN STABLE MAINTENANCE OF RECOMBINANT PLAMIDS IN SALMONELLA VACCINE STRAINS" GENE, ELSEVIER BIOMEDICAL PRESS. AMSTERDAM, NL, vol. 94, no. 1, 28 September 1990 (1990-09-28), pages 29-35, XP002030001 ISSN: 0378-1119
- DEGRYSE E: "Stability of a host-vector system based on complementation of an essential gene in Escherichia coli" JOURNAL OF BIOTECHNOLOGY, ELSEVIER SCIENCE PUBLISHERS, AMSTERDAM, NL, vol. 18, no. 1/2, 1 April 1991 (1991-04-01), pages 29-39, XP002092102 ISSN: 0168-1656
- LUKACSOVICH T ET AL: "NEW APPROACHES TO INCREASE THE EXPRESSION AND STABILITY OF CLONED FOREIGN GENES IN ESCHERICHIA COLI" JOURNAL OF BIOTECHNOLOGY, ELSEVIER SCIENCE PUBLISHERS, AMSTERDAM, NL, vol. 13, no. 4, 1 March 1990 (1990-03-01), pages 243-250, XP000117003 ISSN: 0168-1656
- LUKACSOVICH T ET AL: "A NEW PROCEDURE FOR THE TARGETED INACTIVATION OF ESSENTIAL BACTERIAL GENES" MOLECULAR AND GENERAL GENETICS, SPRINGER VERLAG, BERLIN, DE, vol. 218, no. 2, 1989, pages 364-366, XP002025238 ISSN: 0026-8925
- BOCHNER B R ET AL: "POSITIVE SELECTION FOR LOSS OF TETRACYCLINE RESISTANCE" JOURNAL OF BACTERIOLOGY, vol. 143, no. 2, 1980, pages 926-933, XP009002937 ISSN: 0021-9193
- YANG ET AL.: "HOMOLOGOUS RECOMBINATION BASED MODIFICATION IN ESHERICHIA COLI AND GERMLINE TRANSMISSION IN TRANSGENIC MICE OF A BACTERIAL ARTIFICIAL CHROMSOME" NATURE BIOTECHNOLOGY, NATURE PUBLISHING, US, vol. 15, no. 9, 1 September 1997 (1997-09-01), pages 859-865, XP000770319 ISSN: 1087-0156
- ZHANG YOUMING ET AL: "A new logic for DNA engineering using recombination in Escherichia coli." NATURE GENETICS, vol. 20, no. 2, October 1998 (1998-10), pages 123-128, XP002225129 ISSN: 1061-4036

## Description

### Field of invention

The present invention relates to a method for deletion of antibiotic resistance and /or vector stabilisation in a host cell, as a method of producing vector DNA and/or recombinant protein. It also embraces a transformed host cell, in which a chromosomal essential gene has been deleted and which comprises a vector containing this gene. Further the invention relates to the use of vector DNA produced according to the invention for preparation of a pharmaceutical composition comprising DNA lacking any antibiotic resistance gene.

### Background of the invention.

The stable maintenance of a vector, particularly at high copy number, is important for the preparation of DNA and the protein expressed from it. A cloning vector, such as a plasmid, comprising cloned genes and having been introduced into a host such as a bacteria is easily lost during cultivation of the bacteria. This is because of uncontrolled distribution of the vector to the daughter cells upon cell division. Also, the vector is a metabolic burden to the bacterial host. Bacteria having lost the plasmid generally grow better and take on in numbers compared to the ones harbouring the plasmid.

Accumulation of plasmid-free segregants represents a problem for basic replicon plasmids that are used for cloning and expression purposes, since they do not carry any gene system for an ordered distribution to the host daughter cells. This represents a major industrial problem for maintenance of expression plasmids in the host bacterium during large-scale cultivation. A number of approaches have been tried in order to overcome segregational instability of cloning plasmids.
i) The use of some antibiotic resistance gene inserted into the vector and addition of the corresponding antibiotic to the growth medium represents the most conventional selection pressure for plasmid maintenance. In addition to the economic cost of the antibiotic, a potential environmental hazard from both the antibiotic itself and the resistance gene in the industrial waste is obvious. Appearance of plasmid-free segregants is not prevented totally, since the concentration of the antibiotic used for the plasmid selection often decreases during long-term cultivation as a result of dilution and/or enzymatic degradation by the growing cells.
ii) A natural stabilisation system that is composed of several genes operating in concert has been suggested. The application of this system to stabilise an otherwise dispensable plasmid results in an increased size of the plasmid (Mantile et al., 1999), which could lower the growth rate of the host. This provides a selection for truncated plasmids or plasmid-free segregants that grow faster thus overtaking the culture (Weber et al., 1991).
   A control function in a gene carried by a plasmid has been used to direct a chromosomal gene with self-suicide function. Cells without plasmids do not have the control function and are killed, whereby plasmid stabilisation is created (Boe et al., 1997).
iii) Attempts have been taken to genetically alter an essential gene valS, coding for the protein valyl-tRNA synthetase, in the host cell that makes it dependent on the presence of the plasmid-born corresponding wild-type gene for growth (Nilsson et al., 1986).
   This method is based on a system where the gene valS coding for an essential protein valyl-tRNA synthetase with a temperature sensitive defect is present in the chromosome and the normal wild type allele is present in a plasmid. When temperature is raised the cell can only grow when the vector with the wild type allele is present.
   However, since the chromosomal gene copy is not deleted, although being genetically modified, the frequency of restoration of a wild type chromosomal gene copy as a result of allelic exchange with the plasmid-borne gene is high. Thus, the method in such cases requires a recombination deficient (*recA*⁻) strain, which gives a growth disadvantage for the host strain. However, antibiotic resistance genes are present in the plasmid and are not deleted making the industrial waste hazardous.
iv) Also, in some other cases plasmid stabilisation is done without the use of antibiotic resistance during cultivation.
   For example; deleting the chromosomal copy of an essential Escherichia coli ssb gene and replacing it into a plasmid has been practised (Porter et al., 1990). However, the resistance gene on the plasmid has not been removed.
   Another problem with the plasmids according to these known methods is that the size of the plasmids is rather big. This is due to the presence of the antibiotic resistance gene and the fact that the gene constructions used for creating plasmid stability are rather long. Thus, the insertion of any desired gene to be expressed gives an even larger plasmid. Therefore, there is a risk that the plasmid is not highly stable in the growing culture and that it has a negative effect on growth rate of the host cell.
   USP 5,972,708 describes plasmid stabilisation by a system where a plasmid contains an operon under control of a repressor coded for by a chromosomal gene. The repressor is titrated by binding to this operon. In the chromosome there is another gene, essential for cell growth, under the control of the same repressor. As long as the plasmid is present in the cell the titration effect causes the essential chromosomal gene to be expressed. It is however shut off by repressor blocking if the plasmid and the titrating effect are lost. The resistance gene on the chromosome not being removed, the environmental risk for spreading of the antibiotic resistance gene is still present.

### Summary of the invention

The present invention relates to a method for deletion of antibiotic resistance and /or creation of vector stabilisation in a host cell. According to the present invention a new vector is produced that gives a total selection for host cells that carry this vector. This property is obtained by deleting the chromosomal copy of an essential gene and replacing it into the plasmid. As a result only plasmid carrying cells can grow, making the host totally dependent on the plasmid. Preferably a small gene such as the gene *inf*A is used. The gene *inf*A codes for the translation-initiation factor 1 (IF1) which is a small intracellular and essential factor for cell viability (Cummings et al., 1994). The plasmid does not contain any antibiotic gene and antibiotic selection is thus not necessary, providing considerable advantages during cultivation and elimination of significant environmental concerns. The method is especially advantageous for stabilisation by means of vectors in plant bacteria for transgenic plants to be set out in greenhouses and in nature, where no elimination of the host is envisaged.

The method according to the invention can be used for producing vector DNA and amino acids, peptides or proteins, such as recombinant proteins. The vector DNA may be used for preparing a pharmaceutical composition or vaccine for use in gene therapy. It also embraces a transformed host cell, in which a chromosomal essential gene has been deleted and which comprises a vector containing this gene.

### Detailed description of the invention

The method for deletion of antibiotic resistance and /or creation of plasmid stabilisation according to the invention is characterised in:
a) constructing a vector comprising an antibiotic resistance gene surrounded by a direct repeat sequence gene, which direct repeat gene may be an essential gene
b) in case the repeat sequence gene is not the essential gene then also inserting the essential gene and a suitable promoter for the essential gene in the vector,
c) possibly also inserting a multiple cloning site
d) transfecting a host cell with the vector obtained in a), b) or c)
e) deleting the chromosomal essential gene in the host cell
f) deleting the antibiotic resistance gene in vivo,

Steps a), b) and c) may be done in any order.

A vector consisting of long nucleic acid sequences is less stable than a vector consisting of shorter sequences. Consequently more copies of a vector containing nucleic acid sequences of interest may be obtained with a more stable vector. Moreover, vectors containing long nucleic acid sequences more frequently undergo mutations that may destroy the construction of DNA sequences in the vector. For such reasons, the essential gene should be as small as possible.

The total length of the nucleic acid sequence in the vector also depends on the length of the DNA sequence X of interest that may be inserted into the vector for copying of the DNA sequence X. Further the choice of the essential gene is dependent on the micro-organism i.e. the host intended for the vector. It is therefore not possible to state an optimal length of the essential gene. Generally it is more favourable the shorter the gene is.

Preferably the essential gene is *inf*A coding for translation initiation factor IF1. The coding sequence of the *infA* gene contains only 213 bp. It has turned out that vectors tested, containing the *infA* gene and no antibacterial resistance gene, remain stable. Moreover, there is much room in the vectors for insertion of a desired gene to be expressed and/or for securing that the vector does not become so big as to be a great burden for the host cell and become instable.

When *inf*A is used as the essential gene an essential IF1 (translation-initiation factor 1) is encoded in the vector-carrying host only by the plasmid-born *infA*⁺ gene. Since IF1 is an intracellular and essential protein for cell viability (Cummings et al., 1994), this makes the plasmid highly stable in the culture and the plasmid-free segregants are unable to grow since there is no cross-feeding effect.

By using an essential gene as a selective marker, the gene coding for antibiotic resistance that has been used during the construction of the vector, may be deleted *in vivo* by recombination between the two flanking direct repeat sequences (Rek sequences). This can be done once the essential gene has been inserted. Once the strain with the chromosomal deletion of the essential gene and with the same essential gene in the vector has been constructed there is no need for the antibiotic resistance gene. It is possible to use the essential gene sequence as a repeat sequence on both sides of the antibiotic resistance gene in the vector and omit step b) in the method above.

By the expression " the same essential gene" is understood that substantially the same gene sequence as the essential chromosomal gene sequence is inserted in the vector. Some differences that do not affect the product expressed by the gene may be present such as differences related to the degeneracy of the genetic code or mutationally altered gene products with maintained activity.

When the Rek sequences are chosen from other sequences than the essential gene sequence the following steps may be performed:
a) constructing a vector comprising an antibiotic resistance gene surrounded by a direct repeat sequence, which could be a gene,
b) inserting an essential gene and a suitable promoter for the essential gene and a multiple cloning site in the vector,
c) transfecting a host cell with the vector obtained in b)
d) deleting the essential chromosomal gene in the host cell
e) deleting the antibiotic resistance gene in vivo,
whereby the steps a) and b) may be done in the opposite order.

A suitable promoter allows for transcription of the essential gene. Examples of promoters to be used are the pTrc or the *inf*A promoter.

By the expression "comprising" we understand including but not limited to. Thus, other non-mentioned genes may be present. Especially the vector may carry an inserted gene of interest, which may be multiplied without the need for growth in the presence of antibiotics. Thus, one or more genes X of interest to be copied, produced or expressed by the vector/host system may be inserted into the vector. This may be done at any step in the process but not after the deletion of the antibiotic resistance gene in the vector as there would then be no means for selecting hosts carrying the genes of interest. Preferably a multiple cloning site is inserted in the vector. Also one or more suitable promoters may be included for transcription of the gene(s) X of interest such as the rrnB promoter.

The chromosomal essential gene may be deleted and replaced in E. coli by the method described and selection for the gene replacement may be performed as described by Link et al. (1997). It is also possible to delete the chromosomal gene by the M13 mp (Blum et al. 1989) plasmid method or by PCR amplification followed by transformation of linear DNA.

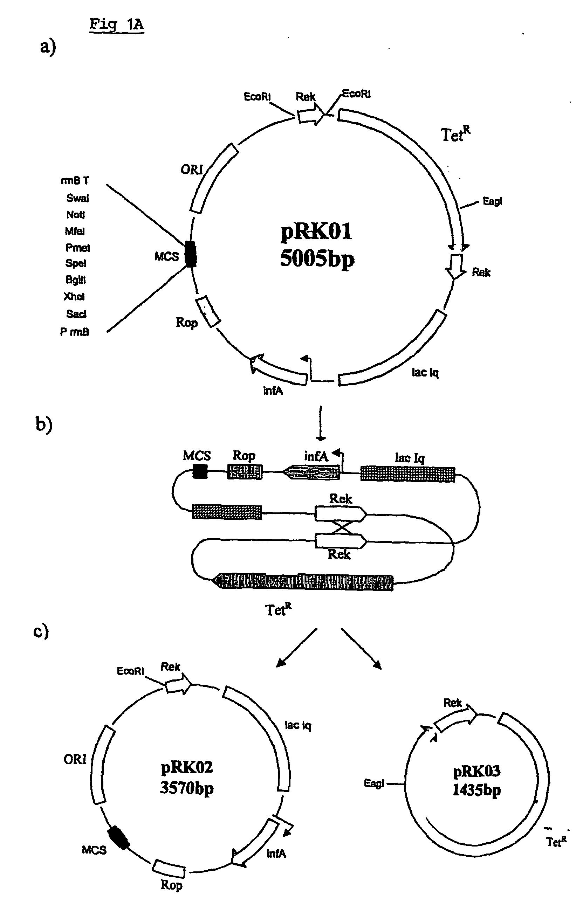

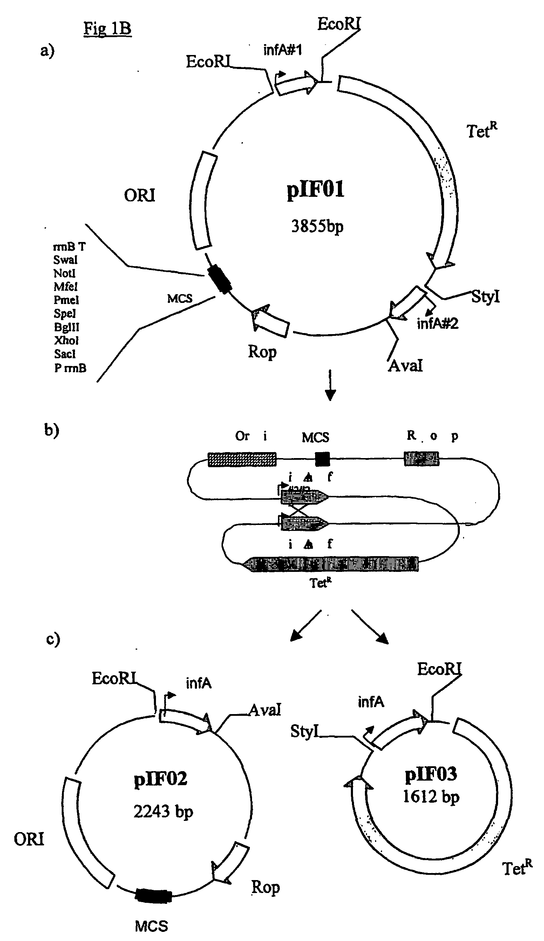

In most applications of the invention the antibiotic resistance gene is eliminated by homologous recombination in vivo between two flanking direct repeats, as known in the art, whereby one of the direct repeat sequences is left in the vector. By repeat sequence we understand a DNA sequence that repeat in the same direction. The repeat sequence may be any sequence that can be deleted together with the antibiotic resistance gene. The direct repeat sequence may be the essential gene e.g. gene *inf*A (Fig.1B) or any direct repeat as exemplified by Rek in Fig.1A, Preferably the repeat sequence is at least 150 base pairs long and is created by PCR amplifying a sequence downstream of the antibiotic resistance gene and then insert it upstream thereof. It is also possible to synthesise a Rek sequence and use restriction enzymes and polylinkers to create the two Rek sites.

Selection for deletion of the antibiotic resistance gene may not be needed, since up to about 50% of non-selected hosts in the long run would lose it spontaneously by the recombination process between the direct repeats. This does however depend on the intended use of the end product of the process.

In details, according to one preferred embodiment when the essential gene is the *infA* and is not the Rek sequence, the following steps may be performed:
1. The ampicillin resistance gene *bla* is deleted from the plasmid pBR322 giving pBR322-Δ Amp. This may be done by the long template PCR technique or by the use of restriction enzymes.
2. A direct repeat sequence Rek is arranged on both sides of the tetracycline resistance gene, *tet* gene, e.g. by PCR amplifying a sequence downstream of the *tet* gene, specially by using the primer sequences Rek 1 and 3 in table II, and then cloning the PCR amplified DNA into the plasmid upstream of the *tet* gene. The gene *infA* is cloned into the commercial pTrc-99A plasmid after the pTrc promoter giving the plasmid pTrc99A-*infA*.
3. A fragment containing the pTrc-promoter and the *infA* gene is cut out of the plasmid pTrc99A-*infA*.
4. Blunt ends are created on this fragment and the fragment is cloned into the pRK plasmid by blunt end ligation, a fragment comprising a multiple cloning site (MCS) and the promoter for rrnB is ligated into this plasmid, giving plasmid pRK01.
5. The *inf*A structural gene is deleted from pMOS*Blue*-*infA* giving plasmid pMOS*Blue-ΔinfA*.
6. The *ΔinfA* fragment (with 420 bp upstream and 400 bp downstream sequences of the *infA* structural gene) is subcloned into the gene replacement plasmid pK03 according to Link et al. (1997) giving plasmid pK03-Δ*infA*.
7. An E coli strain e.g. MG 1655 is transformed with the plasmid pRK01 and with the plasmid pK03-Δ*infA*.
8. Selection for gene replacement is made preferably as described by Link et al. (1997) giving a strain such as PF1A having a chromosome deletion *ΔinfA* and the plasmid PRK01 having a repeat sequence such as Rek on both sides of the tet gene.
9. Confirmation of chromosomal deletion of *infA* may preferably be made by using primers that are specific for chromosomal sequences on each side of *infA* and PCR amplification of this part of the chromosome and sequenate.
10. If desired, insertion of gene X into plasmid can be done followed by transformation into an appropriate strain, such as PF1A, and selection of the plasmid using tetracycline resistance.
11. Strains having undergone homologues recombination and harbouring plasmid pRK02, with or without gene X, giving loss of the tet gene may be selected by fusaric acid.
12. As a model gene for X the ampicillin resistance (*bla* gene) can be used. This gene can be inserted using selection for ampicillin into PRK01 or pRK02 giving pRK-amp in strain PF1A. This strain with pRK-amp may be used to check plasmid stability in the absence of ampicillin selection. Alternatively the *bla* gene, being a model for gene X, can be inserted as described under note 9 above.
13.Several generations of plasmid harbouring bacteria may be cultivated without ampicillin followed by a check for the presence of the bla gene in the colonies that originate from individual bacteria.

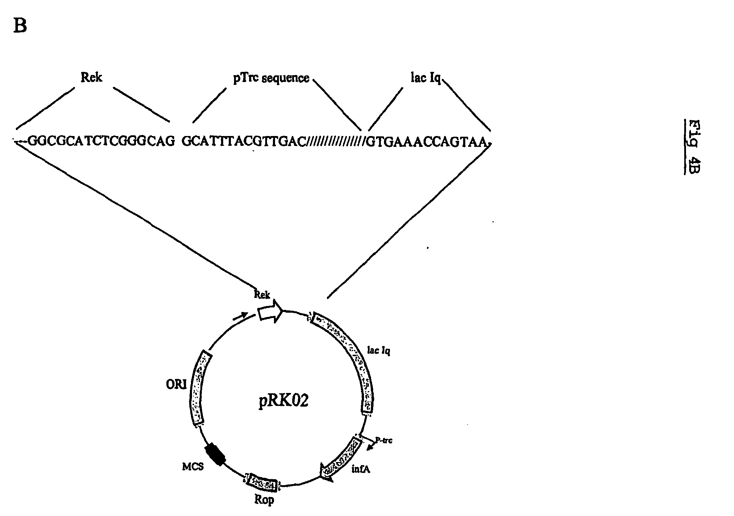

The direct repeat can be the essential gene itself. The two repeats promote homologous recombination leading to precise deletion of the resistance gene (Fig. 1 and Fig. 4B).

With this technique there will be no other Rek sequence present in the end product after recombination but only one copy of the essential gene repeat sequence. In this way the vector will be smaller compared to when the essential gene is inserted into the vector on some other place than as a repeat sequence on both sides of the antibiotic resistance gene.

When the essential gene is used as a Rek sequence a method according to the invention may comprise the steps of:
a) constructing a vector comprising an antibiotic resistance gene surrounded by a direct repeat sequence gene consisting of an essential gene, and comprising at least one appropriately located suitable promoter for the essential gene and a multiple cloning site
b) transfecting a host cell with the vector obtained in a)
c) deleting the chromosomal gene in the host cell
d) deleting the antibiotic resistance gene in vivo,
whereby the steps a) and b) may be done in the opposite order.

When the *inf*A gene is used as an essential gene and as a direct repeat sequence, the following detailed steps may be performed:
1. The ampicillin resistance gene *bla* is deleted from the plasmid pBr322 giving pBR322-Δ Amp.
2. The *inf*A gene and it's promoter is cloned into the plasmid pBR322-Δ Amp upstream of the *tet* gene.
3. Another copy of the *inf*A gene is inserted downstream of the *tet* gene in plasmid pBR322-Δ Amp.
4. A fragment comprising a multiple cloning site (MCS) and the promoter for rrnB is ligated into this plasmid, giving plasmid pIF01.
5. The *inf*A structural gene is deleted from pMOS*Blue*-*infA* giving plasmid pMOS*Blue-ΔinfA*.
6. The *ΔinfA* fragment (with 420 bp upstream and 400 bp downstream sequences of the *infA* structural gene) is subcloned into the gene replacement plasmid pK03 according to Link et al. (1997) giving plasmid pK03-Δ*infA*.
7. An E. coli strain e.g. MG 1655 is transformed with the plasmid pIF01 and with the plasmid pK03*-ΔinfA*.
8. Selection is made for gene replacement preferably as described by Link et al. (1997) giving a strain such as PF1A having a chromosome deletion *ΔinfA* and the plasmid pIF01 having a repeat sequence such as *infA* on both sides of the tet gene.
9. Chromosomal deletion of *inf*A is confirmed preferably by using primers that are specific for chromosomal sequences on each side of *infA* together with PCR amplification of this part of the chromosome and sequence determination.
10. Strains having undergone homologue recombination and harbouring plasmid pIF02 without the tet gene are selected by fusaric acid.
11. Stability may be confirmed by ligating the ampicillin resistance *bla* gene into the plasmid pIF02 and E.coli strain PF1A.
12. Cultivation of several generations without ampicillin and control of the presence of the *bla* gene in the colonies may be done.

When the essential gene is used as a repeat sequence and when the antibiotic resistance gene is deleted by homologous recombination, there is need for a suitable promoter to be present only in repeat sequence that will be present in the surviving host.

The invention also encompasses a method of maintaining a vector in a host cell, comprising the step of culturing the above-described transformed cell for a time and under conditions sufficient to permit the cell to grow. This is useful in order to get a good production of any product of interest. Such a product may be a protein coded for by an inserted gene or some DNA, e, g, a gene, such as a gene in the plasmid.

As used herein, "cell growth" refers to increasing numbers of cells in a culture medium over time, and may also refer to cell survival where the number of cells does not increase over time, and not decrease over time in a culture medium (steady-state)

The invention also encompasses a method of producing vector DNA with inserted genes, comprising culturing the above-described transformed cell for a time and under conditions sufficient to permit the cell to grow, and isolating vector DNA with inserted genes from the cultured cell.

Such a method according to the invention may be used to produce vector DNA and DNA carrying a therapeutic gene for use in gene therapy and to trigger an immune response by DNA vaccine.

Therefore, the invention also encompasses the use of a DNA produced according to the invention for preparation of a pharmaceutical composition for gene therapy such as a vaccine.

In this case the gene of interest is expressible in a mammalian, preferably a human, cell. Examples of such genes are known in the art. If desired, the gene of interest will not be expressed in the host strain. Where the host strain is a bacterium, this can be achieved by not including a bacterial promoter operatively associated with the gene of interest.

The invention also encompasses a method of producing biological metabolites, such as amino acids, as well as peptides or proteins, comprising culturing the above- described transformed host cell with the vector inserted gene X appropriately chosen for a time and under conditions sufficient to produce these products. Preferably, the method further comprises isolating these products.

This method may be especially useful for the preparation of biological metabolites such as amino acids and peptides that are not easily produced synthetically or by other methods.

The protein may be any protein, such as a recombinant protein and a protein of therapeutic benefit to a human or an animal.

The invention may also be used for high expression of inserted genes in the vector. If such genes code for enzymes, which synthesise and overproduce bioorganic metabolites of intermediary metabolism such metabolites, many of which normally are difficult to synthesise by organic chemical methods, can instead be produced by large-scale cultivation of the bacterial strain.

The invention also relates to a transformed host cell in which a chromosomal essential gene with no cross-feeding effect has been deleted and which comprises a vector containing the essential gene and possibly also a gene X of interest. The cell or the vector doesn't comprise any gene for antibacterial resistance. The essential gene can be under control by a suitable promoter, including its natural promoter.

### Host Cells Useful According to the Invention.

The invention is applicable to bacteria with plasmid or virus that can infect animal cells such as mammalian cells, eucaryotic and prokaryotic cells, such as and insect cells, plant cells, fungi. The invention can also be applied to yeast, and most strains of bacteria, for example, Gram positive and Gram negative bacterial strains.

In one embodiment, the host cell is a bacterial cell that may be either gram negative or positive, for example, E. coli, such as E. coli strains DH5α. MG1655 and PF1A but also others, Salmonella e.g., S. Typhimurium, Bacillus, Streptomyces and Lactobacillus. The E. Coli strains mentioned above grow on all types of media.

Gram negative bacteria useful according to the invention include but are not limited to E. coli and Salmonella.

Gram positive species useful according to the invention include but are not limited to Bacillus, Streptomyces, Lactobacillus and Lactococcus, for which high copy number plasmids already exist. Examples of plasmids useful according to the invention in Lactococcus are pNZ2123 and pIL253 (Simon et al. 1988). Examples of plasmids useful according to the invention in Bacillus are pC 194, pUB110 and pT181.

Yeasts are useful according to the invention, as high copy number plasmids are maintained in yeasts. Examples of such plasmids include the YRp plasmids (based on autonomously replicating sequences (ARS)) which have copy numbers up to about 100 copies per cell, and the YEp plasmids (based on the 2 micron circle), with a copy number of 50-100 per cell. (See Sikorski, 1993; Ausubel et al., 1994.)

The invention is especially applicable on bacteria with plasmids or viruses that can infect plants, such as Agrobacterium. When plant cells are gene manipulated they are intended to be used in nature or in greenhouses. Therefore, it is especially undesired to use antibiotic resistance for maintenance of vectors in of these host cells. Contrary to bacterial hosts used for production of recombinant proteins or RNA or DNA, plant hosts cannot be destroyed in order to limit the spread of these unpleasant genes.

### Vectors Useful According to the Invention.

Any replicon, cloning vehicle or vector may be used that are compatible with the host to be used.

A plasmid useful according to the invention must have a plasmid origin that can cope with the host. The invention can be utilised advantageously with a plasmid origin of replication that permits replication giving a high copy number of the plasmid in the cell.

Of the frequently used origins of replication, pBR322 (about 20 copies/cell) and PRK02 are useful according to the invention. pUC (at about 200 copies/cell) may also be used. Examples of such plasmids include but are not limited to pBR322 and the pUC series of plasmids as described by Vieira & Messing (1982, Gene, 19(3), 259-268 and Yanisch-Perron et al. (1985, Gene, 33(1), 103-119). Phages may also be used such as phage λ.

Baculovirus may be used for insect cells. For yeasts, yeast expressions vectors, episomal or plasmid vectors, integrating vectors and yeast artificial vectors may be used such as chromosomes YACs.

Vectors suitable for plants are exemplified by Ti plasmids from A. tumefaciens and Ti plasmid derived vector systems.

### Use in gene therapy.

Plasmid DNA produced according to the invention may be used in gene therapy, when the plasmid contains a therapeutic gene. A therapeutic gene is one which is expressible in a mammalian, preferably a human, cell and encodes RNA, DNA or a polypeptide that is of therapeutic benefit to a mammal, preferably a human. Examples of such genes are well known in the art and include but are not limited to the antigens of different sources to be expressed in the mammalian body and produce antigenic substances or vaccines against which antibodies are to be produced. The DNA may code for substances causing different types of allergic reactions such as hay fever, contact allergy etc. It may also code for proteins from infectious micro-organisms such as bacteria, viruses, mycoplasma etc..

The DNA may also be genes that are important for various conditions such as beta.-glucocerebrosidase gene, the Bruton's thymidine kinase gene, genes encoding cytokines, such as TNF, interleukins 1-12, interferons (α, β, γ), Fc receptor, T-cell receptor, and p53. Other examples include genes encoding inhibitors of HIV, e.g., TAT or REV mutants that act as competitive inhibitors of the natural proteins. The plasmid DNA may also include marker genes, such as drug resistance genes, the beta-galactosidase gene, the dihydrofolate reductase gene, and the chloramphenicol acetyl transferase gene.

Such DNA or RNA may be used in vivo or ex vivo where the therapeutic gene encodes a product of physiological importance, such as replacement of a defective gene or an additional potentially beneficial gene function, is expected to confer long term genetic modification of the cells and be effective in the treatment of disease.

Plasmid DNA containing a therapeutic gene is administered using a viral or non-viral mode of in vivo or ex vivo gene therapy. The mode of administration is not critical to the invention, and may include the use of a gene gun for administration of naked DNA, receptor mediated gene therapy, e.g., using liposome/antibody complexes, and viral vectors.

For example, a patient that is subject to a viral or genetic disease may be treated in accordance with the invention via in vivo or ex vivo methods. For example, in vivo treatments, plasmid DNA of the invention can be administered to the patient, preferably in a biologically acceptable solution or a pharmaceutically acceptable delivery vehicle, by ingestion, injection, inhalation or any number of other methods. The dosages administered will vary from patient to patient; a "therapeutically effective dose" will be determined by the level of enhancement of function of the transferred genetic material balanced against any risk or deleterious side effects. Monitoring levels of gene introduction, gene expression and/or the presence or levels of the encoded product will assist in selecting and adjusting the dosages administered. Generally, a composition including a delivery vehicle will be administered in a single dose in the range of 10 ng-100 µg/kg body weight, preferably in the range of 100 ng-10 µg/kg body weight, such that at least one copy of the therapeutic gene is delivered to each target cell.

Ex vivo treatment is also contemplated within the present invention. Cell populations can be removed from the patient or otherwise provided, transfected with a plasmid containing a therapeutic gene in accordance with the invention, then reintroduced into the patient. If an appropriate Rek sequence is introduced to give a direct repeat that flanks the gene that is used for positive selection of the plasmid upon transfection (could be, but not limited to, antibiotic resistance), the recombination between the homologous sequences will give the desired elimination of that selectable gene in the plasmid in the cell.

The cells targeted for ex vivo gene transfer in accordance with the invention include any cells to which the delivery of the therapeutic gene is desired, for example, cells of the immune system such as T-cells, B-cells, and macrophages, hematopoietic cells, and dendritic cells. Using established technologies, stem cells may be used for gene transfer after enrichment procedures. Alternatively, unseparated hematopoietic cells and stem cell populations may be made susceptible to DNA uptake as described herein. If desired, the plasmid may also include sequences, which confer position independent, tissue specific gene expression, as taught in PCT/GB88/00655.

The new construct according to the invention has several advantages. It does not contain any antibiotic resistance so there is no need for using antibiotic for vector maintenance. It allows for cheap cultivation, as selection based on antibiotics is not necessary. The spreading of bacterial genes that give resistance to antibiotics provides an environmental hazard, which is worsened by the release of antibiotics from the cultivation plant as well. Moreover, plasmid-free cells do not accumulate even after an extended period of growth. The method according to the invention is especially useful for deleting antibiotic resistance genes and inserting genes to make recombinant gene products in plant cells.

IF 1 is a small protein consisting of only 71 amino acids. This means that the size of the vector that carries *infA* can be maintained small, and this is the preferred essential gene used according to the invention. The metabolic burden of the plasmid on cellular growth is therefore at a minimum. The small size of the vector allows for insertion of rather large DNA fragments with genes that are desired to be express. The pRK-plasmid constructed in this work is small in size (3570 basepairs). The doubling time of PF1A harbouring pRK plasmid is virtually unaffected by the deletion of *infA* in both rich broth medium and in a defined M9 medium, as compared to the parental strain MG1655 harbouring pRK plasmid (Table III). Furthermore, the cell mass at the end of the cultivation is identical in both strains in both medium (not shown)

The vector containing the *infA* insert being small brings about that it does not hamper growth. Rather the growth is quite normal (see Example 7). Also, the small size of the plasmid and the *infA* gene means that undesired mutations will occur at a very low frequency. In cases when mutation will occur in *infA* and cause inactivation of the gene product (IF1) and disturbed replication of the plasmid, such cells will stop growing being diluted out during the cultivation. The plasmid being small makes it possible to insert rather large genes of interest into the DNA cloning cassette in the plasmid, without serious lowering of the plasmid copy number.

The method can be used to modify existing production strains and plasmids to the described concept. The indicated system should eliminate potential industrial cultivation problems by its total stability and environmental problems by eliminating the risk of antibiotic resistance gene-transfer between bacterial population and the use of antibiotics during fermentation.

The invention presents a strategy to delete the antibiotic resistance gene that is used during the early steps of construction. This could be done since the vector contains a direct repeat (Rek sequences) surrounding the antibiotic resistance gene in the starting vector. Thus, the system of deletion of an essential chromosomal gene, insertion of a corresponding essential gene in a vector together with the elimination of the antibiotic resistance gene according to the invention provide an essential method to eliminate the risk for environmental spreading of an antibiotic resistance gene besides the advantage of total plasmid stability in cultures.

All publications cited herein are incorporated by reference. The invention will now be elucidated by way of examples, which however do not limit the scope of the invention.

### FIGURE LEGENDS

### Figure 1

Strategy outline for the construction of the plasmids:
A) pRK-plasmid
   a) The starting vector plasmid is pRK01. A duplication of the 150 bp downstream sequence of the tetracycline resistance gene (Rek sequence, white arrows) is inserted upstream of this gene to generate a direct repeat flanking the resistance gene.
   b) Homologous recombination between the two Rek sequences, results in two mini-plasmids
   c) One replication deficient plasmid (pRK03) containing the tetracycline resistance gene *(tet)* and a single copy of the Rek sequence, and a second replicative plasmid (pRK02) with *infA*⁺, ori, and a single copy of the Rek sequence.
B) pIF-plasmid.
   a) The starting vector plasmid is pIF01. This plasmid carries a copy of the *infA* gene inserted on both sides of the *tet* gene to generate a direct repeat flanking the resistance gene.
   b) Homologous recombination between the two *infA* sequences, results in two mini-plasmids
   c) One replication deficient plasmid containing the tetracycline resistance gene *(tet)* and a single copy of the *infA* gene sequence (pIF03), and a second replicative plasmid (pIF02) with ori, and a single copy of the *infA* gene.

### Figure 2

DNA analyses confirming deletion of the chromosomal copy of the *infA* gene.
A) The PCR-amplified chromosomal *infA* region for both *infA*⁺ and *ΔinfA* strains on 1% agaros gel stained with ethidium bromide. Lanes 1 and 15: λHindIII DNA marker. Lanes 2,7,10 and 14: *infA*⁺ DNA. Lanes 3-6, 8,9, 11-13: *ΔinfA* DNA.
B) Sequence analyses of the PCR-bands from lane 2 with *infA*⁺ DNA (upper sequence) and lane 3 with *ΔinfA* DNA (lower sequence).

### Figure 3

DNA analyses of pRK-plasmids on 1% agarose gel:
Panel I; linearized pRK plasmids. Lane 1: λHindIII DNA marker. Lane 2: pRK01 (pre-recombination state of the plasmid). Lanes 3 and 4: pRK02 (post-recombination).
Panel II; plasmids as in panel I digested with EagI restriction enzyme, with a single restriction site in the *tet*⁺ gene. Lane 6: pRK01 treated with EagI. Lanes 7 and 8: pRK02 treated with EagI.

### Figure 4

Sequence analysis of pRK plasmids. The sequencing primer Psgs1 target sequence is indicated by an arrow upstream of the Rek sequence:
A) Sequence analysis of pRK01, the pre-recombination state of the plasmid.
B) Sequence analysis of pRK02, the post-recombination state.

### Examples

Materials and methods:
Bacterial strain and plasmids:
Bacterial strains and plasmids used in this work are listed in Table I.

The *E.coli* strain DH5α was used as a recipient for routine transformations of all plasmid constructs. The wild type recombination proficient *E.coli* K12 strain MG1655 was used to construct PF1A (MG1655-D *infA*).

### Media and growth conditions:

Liquid and solid media were based on LB medium (1% Bactotrypton, 0.5% yeast extract, 0.5% NaCl) and M9 medium (Miller, 1992) supplemented with 0.2% glucose and 1mg/l thiamine) as a defined medium. Antibiotics were added to the medium when required to give the following final concentrations: ampicillin 200 mg/l, tetracycline 20 mg/l and chloramphinicol 20 mg/l. The selective LB/sucrose and Tc^{s} plates-were prepared as described previously (Link et al., 1997, Bochner et al., 1980; Maloy and Nunn, 1981)

### DNA manipulation/ purification and transformation:

Restriction enzymes and T4 DNA ligase were from New England Biolab (New England, Beverly, MA., USA). DNA manipulations were performed according to conventional methods (Sambroch et al., 1989) following the manufacturers recommendations. QIAEXII gel extraction kit from QIAGEN (Valencia, CA., USA) was used for gel purification of PCR products. pK enzyme-mix from Amersham Pharmacia biotech (Buckinghamshire, England) was used to create blunt-ended PCR products by cleaving the non-template nucleotide overhangs. JETPREP plasmid mini-preparation kit from Saveen (Malmö, Sweden) was used for all plasmid extractions. The plasmid transformations were done following the CaCl₂ procedure (Sambroch et al., 1989).

### PCR amplification:

The primers used for PCR amplifications are listed in Table II.
Two PCR methods were used in this study: a) Short template PCR amplifications were performed by using Taq DNA polymerase from Perkin-Elmer (Norwalk, CO., USA). The preparation of the PCR cocktail was as described previously (Abdulkarim et al., 1994). b) Long template PCR amplifications were performed by using the ExpandTM Long Template PCR System from Boeringer Mannheim (Mannheim, Germany). The PCR cocktail was made according to the manufacturers description.

### Analysis of plasmids and PCR products:

Plasmids and PCR products were analysed on 1% agarose gel in TBE buffer with ethidium bromide.

### Deletion of the chromosomal copy of infA:

Deletion of the chromosomal copy of the *infA* gene in the *E.coli* strain MG1655 was performed by a gene replacement method using the plasmid pK03 (Table I) and the method, as described previously (Link et al., 1997).

### Deletion of the plasmid borne tetracycline resistance gene:

100 µl of 10⁻¹ and 10⁻² dilutions of an overnight culture of the strain PF1A containing the plasmid PRK01 were plated on Tc^{s} selective plates (Maloy and Nunn, 1981; Bochner et al., 1980). The plates were then incubated for 48 hours at 37°C.

### DNA sequencing:

DNA sequencing was performed by automatic sequencing (CyberGene -Huddinge, Sweden) using a DNA sequencing kit (Perkin-Elmer Biosystems).

### Determination of plasmid stability:

Experiments to measure plasmid stability were performed using strain PF1A transformed with pRK-amp. A single colony was used to inoculate antibiotic free LB medium and a 2ml culture was grown overnight at 37°C. The saturated culture was diluted to 10⁻⁶ in fresh LB medium without antibiotics, and kept at continues culture by similar dilutions into fresh LB medium every 24 hr. 100 µl diluted culture samples were plated on LB plates at various intervals and incubated overnight at 37°C. Colonies were replica plated onto LB plates containing 200 mg/l ampicillin to check for the presence of the plasmid. Plasmid mini-preparations were made from several independent colonies.

### Growth rate measurement:

Growth rates were measured in liquid LB or M9 medium supplemented with 0.2% glucose and 1 mg/l thiamin. From a fresh overnight culture 100 µl was inoculated to 20 ml of the same medium in 300 ml flasks. Cultures were aerated with vigorous shaking at 37°C and growth was followed by measuring the increase in optical density of the culture as a function of time.

### Example 1

### Plasmid construction (pRK01)

### Construction of pBR322-Δ Amp plasmid:

As a first step we wanted to construct a plasmid which is a derivative of pBR322, deleted for the *bla* gene but with same origin of replication (colE 1), a similar copy number and *tet* as a single antibiotic resistance gene. In order to delete the entire *bla* gene encoding ampicillin resistance, we used the long template PCR technique (se Materials and methods) to amplify the plasmid pBR322 but the *bla* gene. The downstream primer AMPus and the primer Psgs1 (Table II) that is complementary to a region upstream of the *bla* gene were used. The resulting linear 3.3 kb fragment (rest pBR322) was treated with the pK enzyme, blunt-end ligated and transformed into competent DH5α. The transformants were plated on LB containing 20 mg/l tetracycline. To confirm deletion of the *bla* gene, tetracycline resistance colonies were screened for their Amp^{s} phenotype on LB plates containing 200 mg/l ampicillin, followed by restriction enzyme analyses of the plasmid pBR322-ΔAmp (not shown):

### Example 2

### Rek fragment:

As a second step we wanted to insert a direct repeat sequence (designated as Rek fragment) Fig. 1A at each side of the tetracycline resistance gene. This sequence should serve as homologous recombination targets to later on promote deletion of the entire *tet* gene on the plasmid by a homologous recombination event between the two identical Rek-sequences. A 150 base-pair long sequence downstream of the *tet* gene was PCR amplified to give the Rek sequence. The same Rek fragment was then cloned into the EcoRI site upstream of the *tet* gene on the plasmid pBR322-ΔAmp.

### Example 3

### Cloning of the infA gene:

We next wanted to clone the *infA*⁺ structural gene into the plasmid. The *infA*⁺ structural gene was PCR amplified from chromosomal DNA by using primers PH1A3 and PH1A2 (Table II) and cloned into the NcoI and SmaI site in MCS to place it under control of the inducible pTrc promoter in plasmid pTrc99A (Table I). The resulting pTrc99A-*infA*⁺ plasmid was next digested using the SphI restriction enzyme. The resulting SphI fragment, which includes *lacI*, the pTrc promoter and *infA*⁺, was treated with a pK enzyme to generate blunt-ends. The *lacI*⁺, *infA*⁺ cassette was subcloned into the plasmid by blunt-end ligation (see strategy outline in Fig. 1A).

### Example 4

### Cloning of MCS:

Two complementary deoxyoligonucleotides (77 nucleotides long) comprising the E.coli rrnB promoter and several restriction sites were synthesized. The two oligomers were then annealed at room temperature and cloned into the Nde I site of the plasmid (Fig. 1A).

### Example 5

### Strain construction

The PF1A *E.coli* strain was constructed in two steps by a combination of the PCR-technique and a gene replacement method. To construct a strain with a deletion for *infA*, an 1120 bp fragment, including the *infA*⁺ structural gene with the surrounding 420 bp upstream and 400 bp downstream region, was PCR amplified and cloned into the pMOS*Blue* cloning vector. The resulting pMOS *Blue-infA* plasmid was next used as a template for a second round of PCR amplification, by using the two primers Deli1 and Deli2 (Table II). These primers are complementary to regions surrounding the structural *infA* gene and were used by the long template PCR method to amplify the rest of the plasmid including the upstream and downstream regions of *infA*, but excluding the structural gene of *infA* itself. The PCR fragment so obtained was ligated giving plasmid pMOS *Blue-ΔinfA* and transformed into DH5α competent cells. After plasmid preparation and digestion with SalI and BamHI, the *ΔinfA* fragment was subcloned into pK03 generating pK03-Δ*infA* (Table I).
As a second step deletion of the chromosomal *infA* gene was desired. A gene replacement method was used for this purpose. To get the gene replacement, strain MG1655 containing the plasmid pRK01 was transformed with plasmid pK03-Δ*infA* and plated at 30°C on LB plates containing both tetracycline and chloroamphenicol (see example 7 below) In this double selection tetracycline selects for PRK01 and chloroamphenicol for pK03-*ΔinfA*. The selection for the gene replacement event was performed as described previously (Link et al., 1997). Accordingly; 1ml LB broth containing both tetracycline and chloramphenicol was inoculated with a single colony from LB-plates incubated at 30°C. The culture was aerated with vigorous shaking at 30°C for 1-2 hours. A sample of 0.1ml of the culture was plated on LB plates containing tetracycline and chloramphenicol for double selection. The plates were incubated at 43°C overnight. 1-5 colonies from these plates were picked into 1ml LB broth and serially diluted. A sample of 0.1ml of each dilution was plated on LB plates containing 5%(wt/vol.) sucrose and incubated at 30°C overnight. Single colonies were re-streaked on LB/sucrose plates, then screened for pK03 excision from the chromosome by screening for a chloramphenicol sensitive phenotype.

### Example 6

### Confirmation of the deletion of chromosomal infA:

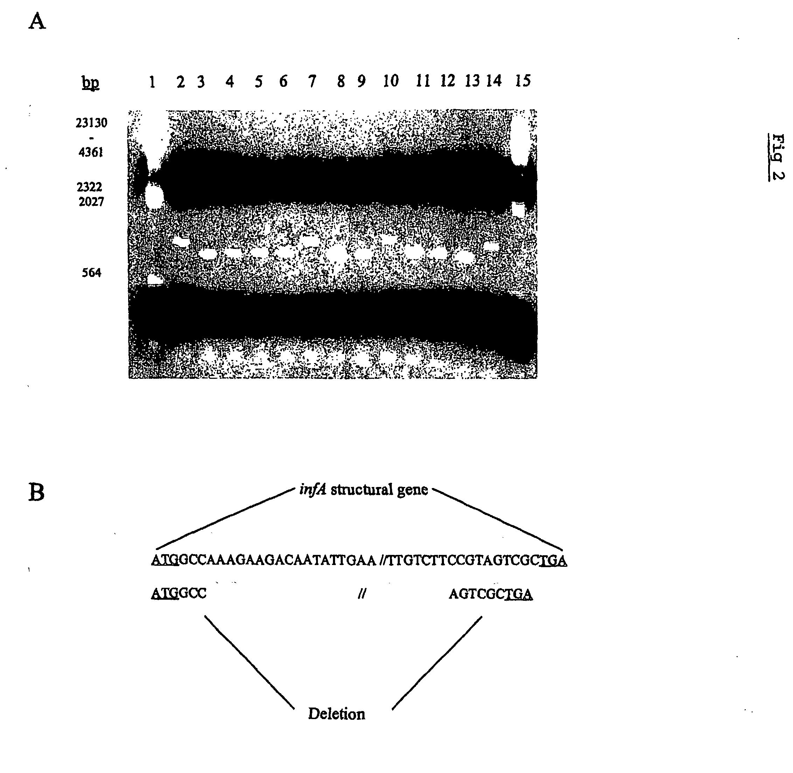

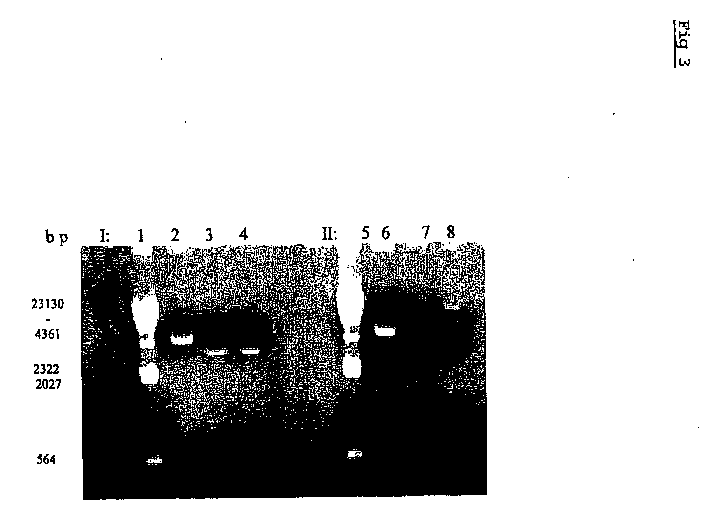

Bacteria with the plasmid pRK01 carrying the structural *infA*⁺ gene and a *ΔinfA* chromosomal deletion (PF1A) should be apparently stable with respect to plasmid maintenance in the absence of tetracycline selection. The same plasmid should be lost with a high probability in a strain with an *infA*⁺ genetic background. After selection on LB/sucrose plates for the excision of pK03 (gene replacement plasmid) from the chromosome in PF1A (Link et al., 1997), chloramphenicol sensitive colonies were streaked on LB plates without tetracycline, followed by 3-4 times of re-streaking on non-selective plates. Single colonies from each streaking after the 3^{rd} or 4^{th} round were screened for the plasmid-born tetracycline resistance phenotype. All colonies maintained their Tet^{r} phenotype. Finally, the chromosomal *infA* region of both Tet^{r} colonies and the parental strain (MG1655) were PCR amplified, using primers PTV5'and PH1A2. The data presented in Fig. 2A show that the PCR band from several Tet^{r} colonies is about 300bp smaller than that of the PCR band from the parental strain. This difference is in a good agreement with the size of the *infA* structural gene. Furthermore, DNA sequencing of the two bands confirmed the expected site and the size of the deletion (Fig. 2B).

### Example 7

### Deletion of the tetracycline gene from the plasmid:

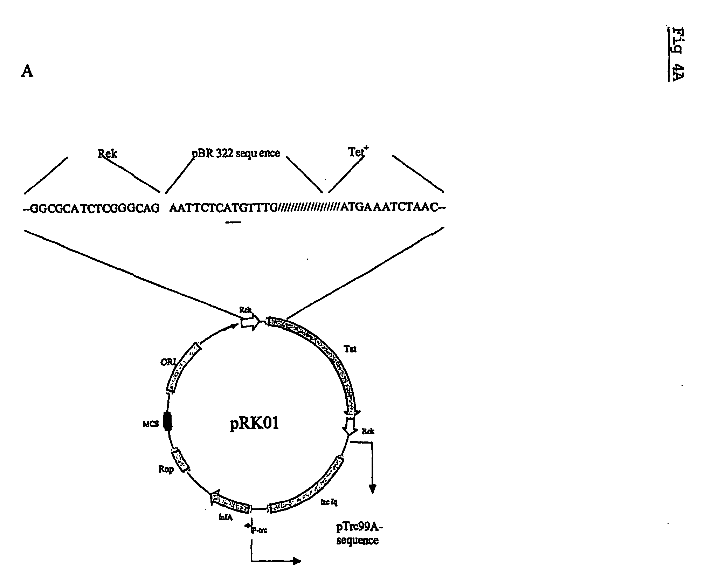

The deletion of the plasmid borne *tet* gene was performed on selective plates
containing 2 mg/ml fusaric acid (see Materials and Methods)
We next wanted to confirm that the deletion of the *tet* gene on the plasmid is caused during growth by homologous recombination in vivo between the two directly repeated Rek sequences. Data presented in Fig. 3 suggest that the pRK02 plasmids from Tet^{s} cells (post-recombinational state of the plasmid: lanes 3 and 4) is about 1000bp smaller than the pRK01 plasmid (lane 2) from Tet^{r} cells (prior to the recombination). Furthermore, the DNA sequence of the band in the post-recombination state (pRK02) shows that the plasmid contains only one Rek sequence. The sequence bypasses the tetracycline gene and continues into the *lacI* sequence of the plasmid pRK02 (Fig. 4B). As a comparison, for the sequence in PRK01 prior to recombination, the sequence continues through the *tet* gene, and the *tet* is still surrounded by the two Rek sequences (Fig. 4A).

### Example 8

### Stability test and growth rate:

To test plasmid stability, the ampicillin resistance (*bla*) gene from pBR322 was used as a model for an inserted gene if interest. It was cloned into the multiple cloning site (MCS) of pRK02, giving pRK-amp and transformed into PF1A and its parental strain MG1655. After growth in the absence of ampicillin for several generations, plasmid maintenance was determined by replica plating from LB-plates to ampicillin-plates. If the plasmid is stable in the PF1A strain the colonies should remain ampicillin resistant. After growth for 120 generations in liquid culture the stability of the pRK-amp plasmid in PF1A in the absence of antibiotic was 100%. As a comparison, more than 90% of the parental MG1655 bacteria, transformed with pRK-amp had lost the plasmid after 50 generations. Such stability was obtained irrespective of if the strain was grown in minimal or broth medium. Furthermore, the doubling time of the strain with the stabilised plasmid was indistinguishable from the parental strain MG1655 in both media (Table III) and the cell densities in stationary phase were the same for both strains in both media.

### References

Abdulkarim, F., Liljas, L., and Hughes, D., 1994. Mutations to kirromycin resistance occur in the interface of domain I and III of EF-TuGTP. *FEBS Letters* 352: 118-122.
Ausubel et al., 1994.Yeast Cloning Vectors and Genes, SectionII, Unit 13.4, Current Protocols in Molecular Biology,
Blum, P; Holzchu, D; Kwan, H-S; Riggs, D &Artz, S. 1989. Gene replacement and retrieval with recombinant M13mp bacteriophages. *J. Bacteriol*. 171: 538-546.
Boe, L., Gerdes, K., and Molin, S. 1987. Effects of gene exerting growth inhibition and plasmid stability on plasmid maintenance. *J. Bacteriol*. 169:4646-4650.
Bochner, B. R., Huang, H. C., Schieven, G. L., and Ames, B. 1980. Positive selection for loss of tetracycline resistance. *J. Bacteriol*. 143: 926-933.
Cummings, H., and Hershey, J. W. B. 1994. Translation initiation factor IF1 is essential for cell viability in E.coli. *J. Bacteriol*. 176: 198-205.
Link, J. A., Phillips, D., and Church, G. M. 1997. Methods for generating precise deletion and insertions in the genome of Wild-type E.coli: Application to open reading frame charaterization. *J. Bacteriol*. 179: 6228-6237.
Maloy, S., and Nunn, W. 1981. Selection for loss of tetracycline resistance by E.coli. *J. Bacteriol.* 145: 1110-1112.
Mantile, G., Fuch, C., Cordella-Miele, E., Peri, A., Mukherjee, A. B., and Miele, L., 1999. Stable, long-term bacterial production of soluble, dimeric, disulfide-bonded protein pharmaceuticals without antibiotic selection.
*Biotechnol. Prog.* 16:17-25
Miller, J. F., 1992. A short course in Bacterial Genetics. A laboratory manual and handbook to E. coli and related bacteria. Cold Spring Harbor Laboratories.
Nilsson, J. and Skogman, S. G. 1986. Stabilization of E.coli tryptophan production vectors in continuous cultures: A comparison of three different systems. *Bio Technology* 4:901-903.
Nordström, K. 1989. Mechanisms that contribute to the stable segregation of plasmid. *Annu. Rev. Genet*.23: 37-69.
Porter et al., "Use of the Escherichia coli ssb gene to prevent bioreactor takeover by plasmidless cells." *Bio*/*Technology* 8; 47-51 (1990)
Ronald, D. P., Stuart, B., Sachin, P., and Alfred, C. 1990. Use of the E.coli SSB gene to prevent bioreactor takeover by plasmidless cells. *Bio Technology* 8: 57-51.
Sambrook, Fritsch, Maniatis. 1989. Molecular Cloning: A laboratory manual. Sikorski, 1993 "Extrachromosomal cloning vectors of Saccharomyces cerevisiae", in Plasmids, A Practical Approach, Ed. K. G. Hardy, IRL Press, Simon et al., Biochimie 70:559, 1988
Vieira & Messing 1982, Gene, 19(3), 259-268
Weber, A. E., Yu, P., San, K. Y. 1991. The errect of the partition locus on plasmid stability and expression of a prolonged chemostat culture. *J. Biotech*, 18: 141-152.
Yanisch-Perron et al. 1985, Gene, 33(1), 103-119

**Table I.**

| Plasmids and bacterial strains. | | |
|---|---|---|
| **Plasmid** | **Genotype** | **Reference or source** |
| pBR322 | *bla*⁺, *tet*⁺ | Amersham Pharmacia Biotech |
| pBR-ΔAmp | *tet*⁺ | This work |
| PMOS*Blue* | *bla*⁺, *lacZ*, fl origin | Amarsham Pharmacia Biotech. |
| PMOS*Blue-infA* | *bla*⁺, *lacZ*, fl origin, *infA(*with 420 bp upstream and 400 bp dounstream sequences of the *infA* structural gene) | This work |
| PMOS*Blue-ΔinfA* | as PMOS*Blue-infA* (with *-ΔinfA* structural gene) | This work |
| pK03 | *cat*, M13 ori, *sacB, repA(ts)* | Link et al., 1997 |
| pK03-*ΔinfA* | *cat,* M13 ori, *sacB, repA(ts) ΔinfA* | This work |
| pTrc99A pTrc99A-*infA* | *bla*⁺, *lacIq*, pTrc promoter. *bla*⁺, *IacIq*, pTrc-*infA*⁺ (structural gene) | Amarsham Pharmacia Biotech This work |
| pRK01 | *tet*⁺, duplicated Rek sequence, *infA*⁺, *lacIq*, MCS, *rop,* pBR ori. | This work |
| pRK02 | *infA*⁺, *lacIq*, MCS, *rop,* Rek sequence, pBR ori. | This work |
| pRK-Amp | As pRK02 with *bla* ⁺(structural gene cloned into MCS) | This work |
| pIF01 | *tet*⁺, duplicated *infA* gene, MCS, *rop,* pBR ori. | This work |
| pIF02 | *infA*⁺, MCS, *rop*, pBR ori. | This work |
| pLF-Amp | As pIF02 with *bla* ⁺(structural gene cloned into MCS) | This work |

| **Bacterial strains** | | |
|---|---|---|
| DH5α | *SupE44 Δ lacu169 (φ80λαχZΔM15) hsdR recA1 endA1 gyrA96 thi-1 relA1* | |
| MG1655 | Wild type strain | Wild type strain |
| PF1A | MG1655-Δ *infA* | This work |

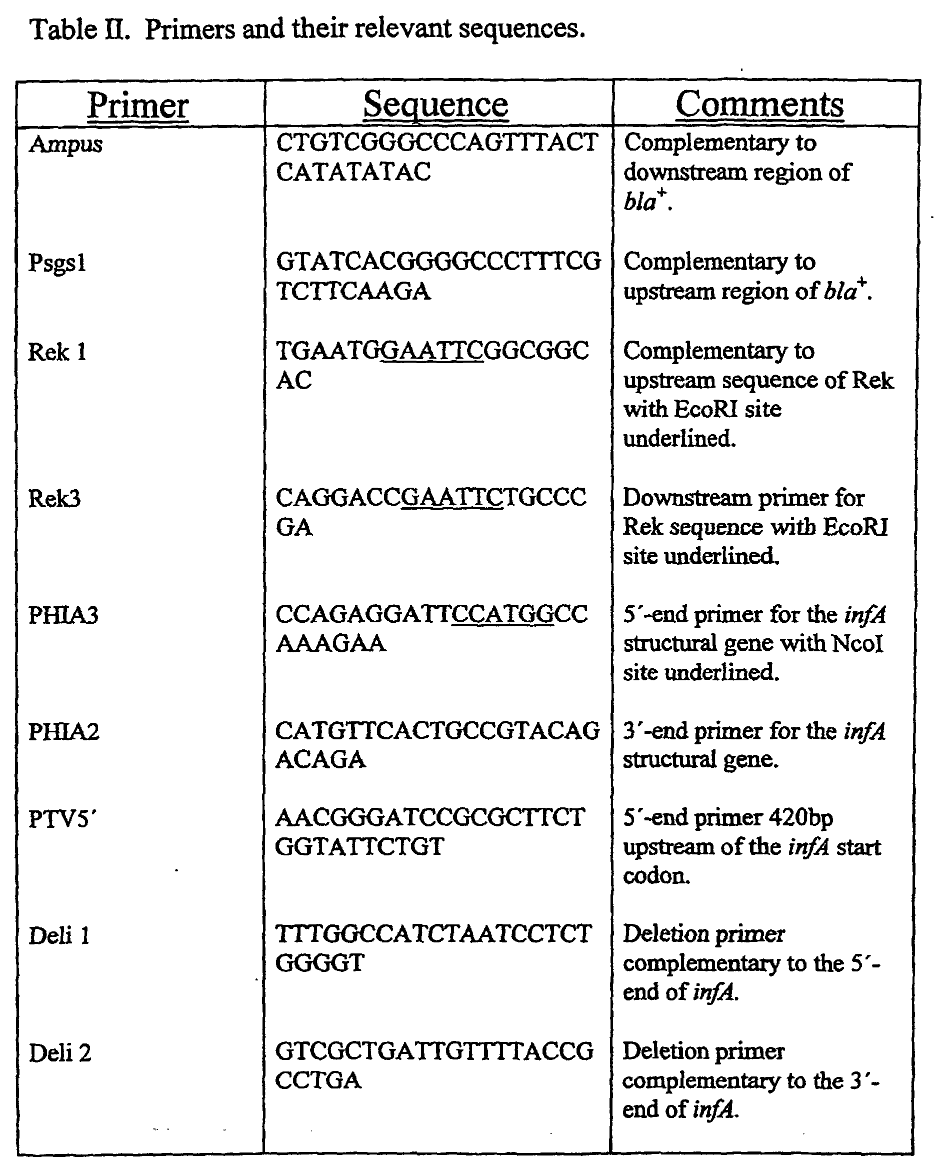

**Table III**

| Doubling time for the PF1A/pRK02 and the wild type MG1655/pRK02 in rich broth medium and a defined M9 medium. | | |
|---|---|---|
| Strain | Doubling time (min) LB | Doubling time (min) M9 |
| PF1A/pRK02 | 31 | 82 |
| MG1655/ pRK02 | 32 | 82 |

## Claims

1. A method for deletion of antibiotic resistance and /or plasmid stabilisation composing the steps of:
a) constructing a vector comprising an antibiotic resistance gene surrounded by a direct repeat sequence gene, which direct repeat gene may be an essential gene
b) possibly also inserting the essential gene and a suitable promoter for the essential gene and a multiple cloning site in the vector,
c) transfecting a host cell with the vector obtained in a) or b)
d) deleting the chromosomal essential gene in the host cell
e) deleting the antibiotic resistance gene in vivo,
whereby the steps a) and b) may be done in the opposite order.

2. A method according to claim 1 comprising the steps of:
a) constructing a vector comprising an antibiotic resistance gene surrounded by a direct repeat sequence gene,
b) inserting an essential gene and a suitable promoter for the essential gene and a multiple cloning site in the vector,
c) transfecting a host cell with the vector obtained in b)
d) deleting the essential chromosomal gene in the host cell
e) deleting the antibiotic resistance gene in vivo,
whereby the steps a) and b) may be done in the opposite order.

3. A method according to claim 1 comprising the steps of:
a) constructing a vector comprising an antibiotic resistance gene surrounded by a direct repeat sequence gene consisting of an essential gene, and comprising at least one suitable promoter for the essential gene and a multiple cloning site
b) transfecting a host cell with the vector obtained in a)
c) deleting the chromosomal gene in the host cell
d) deleting the antibiotic resistance gene in vivo,
whereby the steps a) and b) may be done in the opposite order.

4. A method according to any of claims 1-3, **characterised in that** the essential gene is *inf*A.

5. A method according to any of claims 1-4, **characterised in that** the antibiotic resistance gene is deleted by homologous recombination in vivo.

6. A method according to any of claims 1-5, further comprising the step of inserting a multiple cloning site suitable for introducing or comprising one or more promoters and DNA sequences to be produced or expressed.

7. A method according to any of claims 1-6, further **characterised in that** one or more genes of interest to multiply or express is/are inserted at any step of the process before deleting the antibiotic resistance gene in vivo.

8. A method according to any of claims 1-7, further comprising the step of selection of host cells with antibiotic resistance vector gene deletion.

9. A method of maintaining a vector in a host cell comprising the step of culturing the transformed host cell obtained according to any of claims 1-8 for a time and under conditions sufficient to permit said cell to grow.

10. A method of producing DNA comprising the steps of culturing the transformed host cell obtained according to any of claims 1-8 for a time and under conditions sufficient to permit said cell to grow, and isolating plasmid DNA from said cultured cell.

11. A method of producing one or more amino acids, peptides or proteins comprising the steps of curiuriag the transformed host cell obtained according to any of claims 1-8 for a time and under condidons sufficient to permit said cell to grow, and isolating the amino acids, peptides or proteins.

12. A transformed host cell, **characterised in that**
a chromosomal intracellular essential gene with no cross-feeding effect, preferably *inf*A has been deleted
and **in that** it comprises a vector comprising at least one copy of the same essential gene
and possibly also a gene X of interest
and **in that** it does not comprise any gene for antibiotic resistance.

13. A transformed host according to claim12, **characterised in that** it is any bacterial cell with plasmid or virus that can infect animal cells such as mammalian cells and insect cells, plant cells, fungi such as yeast viruses and bacteria, for example agrobacterium, E. Coli such as E. Coli strains DH5a, MG1655 and PF1A.

14. Use of a vector DNA-obtained from a host-cell produced according to any of claims 1-9, or according to any of claims 11-13 for preparation of a pharmaceutical composition for gene therapy such as a vaccine.

## Patentansprüche

1. Ein Verfahren zur Deletion antibiotischer Resistenz und/oder Plasmidstabilisation, umfassend die Schritte:
a) Konstruieren eines Vektors, der ein antibiotisches Resistenzgen umfaßt, welches von einem direkten Repeatsequenzgen umgeben ist, wobei das direkte Repeatgen ein essentielles Gen sein kann;
b) möglicherweise auch Einschleusen des essentiellen Gens und eines geeigneten Promotors für das essentielle Gen und einer Multiple Cloning Site in den Vektor;
c) Transfizieren einer Wirtszelle mit dem Vektor, der aus a) oder b) erhalten wurde;
d) Deletieren des chromosomalen essentiellen Gens in der Wirtszelle;
e) Deletieren des Antibiotikaresistenzgens in vivo;
wobei die Schritte a) und b) in umgekehrter Reihenfolge vorgenommen werden können.

2. Ein Verfahren gemäß Anspruch 1, umfassend die Schritte:
a) Konstruieren eines Vektors, der ein Antibiotikaresistenzgen, welches von einem direkten Repeatsequenzgen umgeben ist, umfaßt;
b) Einschleusen eines essentiellen Gens und eines geeigneten Promotors für das essentielle Gen und einer Multiple Cloning Site in den Vektor;
c) Transfizieren einer Wirtszelle mit dem Vektor, der aus b) erhalten wurde;
d) Deletieren des essentiellen chromosomalen Gens in der Wirtszelle;
e) Deletieren des Antibiotikaresistenzgens in vivo;
wobei die Schritte a) und b) in umgekehrter Reihenfolge vorgenommen werden können.

3. Ein Verfahren gemäß Anspruch 1, umfassend die Schritte:
a) Konstruieren eines Vektors, der ein Antibiotikaresistenzgen, welches von einem direkten Repeatsequenzgen bestehend aus einem essentiellen Gen umgeben ist, umfaßt, und der mindestens einen geeigneten Promotor für das essentielle Gen und eine Multiple Cloning Site umfaßt;
b) Transfizieren einer Wirtszelle mit dem Vektor, der aus a) erhalten wurde;
c) Deletieren des chromosomalen Gens in der Wirtszelle;
d) Deletieren des Antibiotikaresistenzgens in vivo;
wobei die Schritte a) und b) in umgekehrter Reihenfolge vorgenommen werden können.

4. Ein Verfahren gemäß irgendeines der Ansprüche 1-3, **dadurch gekennzeichnet, daß** das essentielle Gen *infA* ist.

5. Ein Verfahren gemäß irgendeines der Ansprüche 1-4, **dadurch gekennzeichnet, daß** das Anribiotikaresistenzgen durch homologe Rekombination in vivo deletiert ist.

6. Ein Verfahren gemäß irgendeines der Ansprüche 1-5, weiterhin umfassend den Schritt des Insertierens einer Multiple Cloning Site, die geeignet für das Einschleusen oder Umfassen eines oder mehrerer Promotoren und DNA-Sequenzen ist, um sie herstellen oder exprimieren zu können.

7. Ein Verfahren gemäß irgendeines der Ansprüche 1-6, weiterhin **dadurch gekennzeichnet, daß** eines oder mehrere Gene von Interesse zur Vervielfältigung oder Expression bei irgendeinem Schritt des Prozesses vor dem Deletieren des Antibiotikaresistengens in vivo insertiert wird/werden.

8. Ein Verfahren gemäß irgendeines der Ansprüche 1-7, weiterhin umfassend den Schritt der Selektion von Wirtszellen durch Deletion des Antibiotikaresistenzvektorgens.

9. Ein Verfahren des Beibehaltens eines Vektors in einer Wirtszelle, umfassend den Schritt des Kultivierens der transformierten Wirtszelle, die gemäß irgendeines der Ansprüche 1-8 erhalten wurde, für einen Zeitraum und unter Bedingungen, die ausreichen, um besagter Zelle das Wachstum zu ermöglichen.

10. Ein Verfahren zur Herstellung von DNA, umfassend die Schritte des Kultivierens der transformierten Wirtszelle, die gemäß irgendeines der Ansprüche 1-8 erhalten wurde, für einen Zeitraum und unter Bedingungen, die ausreichen, um besagter Zelle das Wachstum zu ermöglichen und um Plasmid-DNA von besagter kultivierter Zelle zu isolieren.

11. Ein Verfahren zur Herstellung eines oder mehrerer Aminosäuren, Peptide oder Proteine, umfassend die Schritte des Kultivierens der transformierten Wirtszelle, die gemäß irgendeines der Ansprüche 1-8 erhalten wurde, für einen Zeitraum und unter Bedingungen, die ausreichen um besagter Zelle das Wachstum zu ermöglichen und um die Aminosäuren, Peptide oder Proteine zu isolieren.

12. Eine transformierte Wirtszelle, **dadurch gekennzeichnet, daß**
ein chromosomales, intrazelluläres, essentielles Gen ohne Cross-Feeding-Effekt, vorzugsweise *inf*A, deletiert wurde und **dadurch gekennzeichnet, daß** es einen Vektor umfaßt, der mindestens eine Kopie des gleichen essentiellen Gens umfaßt,
und möglicherweise auch ein Gen X von Interesse
und **dadurch gekennzeichnet, daß** es nicht irgendein Gen für Antibiotikaresistenz umfaßt.

13. Ein transformierter Wirt gemäß Anspruch 12, **dadurch gekennzeichnet, daß** er irgendeine Bakterienzelle mit einem Plasmid oder Virus ist, der tierische Zellen so wie auch Säugerzellen und Insektenzellen, Pflanzenzellen, Pilze infizieren kann beispielsweise Hefen, Viren und Bakterien, zum Beispiel Agrobacterium, E. coli wie auch die E.coli-Stämme DH5a.MG1655 und PF1.A.

14. Die Verwendung einer Vektor-DNA, welche von einer Wirtszelle erhalten wurde, die gemäß irgendeinem der Ansprüche 1-9 hergestellt wurde, oder gemäß irgendeinem der Ansprüche 11-13 zur Herstellung einer pharmazeutischen Zusammensetzung für die Gentherapie beispielsweise einem Impfstoff.

## Revendications

1. Procédé de délétion de la résistance à un antibiotique et/ou de stabilisation d'un plasmide comprenant les étapes suivantes :
a) la construction d'un vecteur comprenant un gène de résistance à un antibiotique entouré par un gène constitué d'une séquence répétée directe, lequel gène répété direct pouvant être un gène essentiel,
b) éventuellement également l'insertion du gène essentiel et d'un promoteur approprié pour le gène essentiel et d'un site de clonage multiple dans le vecteur,
c) la transfection d'une cellule hôte avec le vecteur obtenu en a) ou b),
d) la délétion du gène chromosomique essentiel dans la cellule hôte,
e) la délétion du gène de résistance à un antibiotique in vivo, les étapes a) et b) pouvant être effectuées dans l'ordre inverse.

2. Procédé selon la revendication 1 comprenant les étapes suivantes :
a) la construction d'un vecteur comprenant un gène de résistance à un antibiotique entouré par un gène constitué d'une séquence répétée directe,
b) l'insertion d'un gène essentiel et d'un promoteur approprié pour le gène essentiel et d'un site de clonage multiple dans le vecteur,
c) la transfection d'une cellule hôte avec le vecteur obtenu en b),
d) la délétion du gène chromosomique essentiel dans la cellule hôte,
e) la délétion du gène de résistance à un antibiotique in vivo, les étapes a) et b) pouvant être effectuées dans l'ordre inverse.

3. Procédé selon la revendication 1 comprenant les étapes suivantes :
a) la construction d'un vecteur comprenant un gène de résistance à un antibiotique entouré par un gène constitué d'une séquence répétée directe, consistant en un gène essentiel et comprenant au moins un promoteur approprié pour le gène essentiel et un site de clonage multiple,
b) la transfection d'une cellule hôte avec le vecteur obtenu en a),
c) la délétion du gène chromosomique essentiel dans la cellule hôte,
d) la délétion du gène de résistance à un antibiotique in vivo, les étapes a) et b) pouvant être effectuées dans l'ordre inverse.

4. Procédé selon l'une quelconque des revendications 1 à 3, **caractérisé en ce que** le gène essentiel est *inf*A.

5. Procédé selon l'une quelconque des revendications 1 à 4, **caractérisé en ce que** le gène de résistance à un antibiotique est supprimé par recombinaison homologue *in vivo*.

6. Procédé selon l'une quelconque des revendications 1 à 5, comprenant en outre l'étape consistant à insérer un site de clonage multiple approprié pour introduire, ou comprenant, un ou plusieurs promoteurs et les séquences d'ADN à produire ou exprimer.

7. Procédé selon l'une quelconque des revendications 1 à 6, **caractérisé en outre en ce que** un ou plusieurs gènes d'intérêt à multiplier ou à exprimer est/sont insérés lors d'une étape quelconque du processus avant de supprimer le gène de résistance à un antibiotique in vivo.

8. Procédé selon l'une quelconque des revendications 1 à 7, comprenant en outre l'étape de sélection des cellules hôtes présentant une délétion du gène vecteur d'une résistance à un antibiotique.

9. Procédé de maintien d'un vecteur dans une cellule hôte comprenant l'étape consistant à cultiver les cellules hôtes transformées obtenues conformément à l'une quelconque des revendications 1 à 8 pendant une durée et dans des conditions suffisantes pour permettre la croissance de ladite cellule.

10. Procédé de production d'ADN comprenant les étapes consistant à cultiver la cellule hôte transformée obtenue conformément à l'une quelconque des revendications 1 à 8 pendant une durée et dans des conditions suffisantes pour permettre la croissance de ladite cellule et à isoler l'ADN plasmidique à partir de ladite cellule cultivée.

11. Procédé de production d'un ou de plusieurs acides aminés, peptides ou protéines comprenant les étapes consistant à cultiver les cellules hôtes transformées obtenues conformément à l'une quelconque des revendications 1 à 8 pendant une durée et dans des conditions suffisantes pour permettre la croissance de ladite cellule, et à isoler les acides aminés, les peptides ou les protéines.

12. Cellule hôte transformée, **caractérisée en ce que**
un gène chromosomique intracellulaire essentiel ne présentant pas d'effet en culture croisée, de préférence *inf*A, a été supprimé,
et **en ce qu'**il comprend un vecteur comprenant au moins un exemplaire du même gène essentiel
et éventuellement également un gène X d'intérêt
et **en ce qu'**il ne comprend aucun gène de résistance à un antibiotique.

13. Hôte transformé conformément à la revendication 12, **caractérisé en ce qu'**il s'agit d'une cellule bactérienne quelconque contenant un plasmide ou un virus susceptible d'infecter des cellules animales comme des cellules de mammifère et des cellules d'insecte, des cellules végétales, des fungi comme la levure, des virus et des bactéries, par exemple des agrobactéries, des souches d'E. coli, comme les souches DH5a, MG1655 et PF1A.

14. Utilisation d'un ADN vecteur obtenu à partir d'une cellule hôte produite conformément à l'une quelconque des revendications 1 à 9, ou conformément à l'une quelconque des revendications 11 à 13 pour la préparation d'une composition pharmaceutique destinée à une thérapie génique, notamment un vaccin.


### SEQUENCE LISTING

<110> Isaksson, Leif
Hägg, Peter Jörgen
Abdulkarim, Farhad

<120> New method

<130> 62870

<140>
<141>

<160> 9

<170> PatentIn ver. 2.1

<210> 1
<211> 28
<212> DNA
<213> Artificial Sequence

<220>
<223> Description of Artificial Sequence: Complementary to downstream region of bla+ in PBR322

<400> 1
   ctgtcgggcc cagtttactc atatatac 28

<210> 2
<211> 28
<212> DNA
<213> Artificial Sequence

<220>
<223> Description of Artificial Sequence: Complementary to upstream region of bla+ in PBR322

<400> 2
   gtatcacggg gccctttcgt cttcaaga 28

<210> 3
<211> 20
<212> DNA
<213> Artificial Sequence

<220>
<223> Description of Artificial Sequence: Complementary to upstream for Rek sequence with EcoRIi site in PBR322

<400> 3
   tgaatggaat tcggcggcac 20

<210> 4
<211> 20
<212> DNA
<213> Artificial Sequence

<220>
<223> Description of Artificial Sequence: Downstream primer for Rek sequence with EcoRIi site in PBR322

<400> 4
   caggaccgaa ttctgcccga 20

<210> 5
<211> 24
<212> DNA
<213> Artificial Sequence

<220>
<223> Description of Artificial Sequence: 5'-end primer for the infA structural gene from E .coli MG1655

<400> 5
   ccagaggatt ccatggccaa agaa 24

<210> 6
<211> 24
<212> DNA
<213> Artificial Sequence

<220>
<223> Description of Artificial Sequence: 3'-end primer for the infA structural gene from E. coli MG1655

<400> 6
   catgttcact gccgtacaga caga 24

<210> 7
<211> 28
<212> DNA
<213> Artificial Sequence

<220>
<223> Description of Artificial Sequence:Description of Artificial Sequence : 5'-end primer 420 bp upstream of the infA start codon from E.coli MG1655

<400> 7
   aacgggatcc gcgcttctgg tattctgt 28

<210> 8
<211> 24
<212> DNA
<213> Artificial Sequence

<220>
<223> Description of Artificial Sequence: Deletion primer complementary to the 5'-end of infA from E. coli MG1655

<400> 8
   tttggccatc taatcctctg gggt 24

<210> 9
<211> 24
<212> DNA
<213> Artificial Sequence

<220>
<223> Description of Artificial Sequence: Deletion primer complementary to the 3'-end of infA from E. coli MG1655

<400> 9
   gtcgctgatt gttttaccgc ctga 24